# EUROPÄISCHE PATENTANMELDUNG

(11) **EP 0 978 267 A2**
(43) Veröffentlichungstag der Anmeldung: **09.02.2000**
(21) Anmeldenummer: 99114689.5
(22) Anmeldetag: 27.07.1999
(51) Int. Cl.: A61G 9/02

(54) **Verfahren und Vorrichtung zum Reinigen und Desinfizieren von Gefässen**

(30) Priorität: 03.08.1998 DE 19834985; 21.08.1998 DE 19838180
(71) Anmelder: BHT Hygiene Technik GmbH, 86316 Friedberg/Derching (DE)
(72) Erfinder: Biermaier, Hans, 86316 Derching (DE); Zanatta, Maurizio, 86153 Augsburg (DE)
(74) Vertreter: von Bülow, Tam, Dr.

(57) **Zusammenfassung**

Bei der Vorrichtung zum Reinigen von Gefäßen - insbesondere Steckbecken, Urinflaschen und Absaugflaschen - werden bei zunächst geöffnetem Absperrorgan (34) zu reinigende Gegenstände (4, 5) entleert und vorgespült. Anschließend erfolgt bei geschlossenem Absperrorgan (34) eine Reinigung bzw. Desinfizierung im Umwälzbetrieb.

## Beschreibung

Die Erfindung betrifft ein Verfahren gemäß dem Oberbegriff des Patentanspruches 1 und eine Vorrichtung gemäß dem Oberbegriff des Patentanspruches 5 und bezieht sich auf das Reinigen von Gefäßen, insbesondere Steckbecken, Urinflaschen und Absaugflaschen.

Die DE 37 09 020 A1 beschreibt eine Steckbecken-Spülmaschine, bei der die Steckbecken zunächst entleert und dann vorgespült werden, wobei kontinuierlich frische Reinigungsflüssigkeit zugeführt und verunreinigte Reinigungsflüssigkeit abgeführt wird. Nach der Vorspülphase wird ein Abfluß der Spülmaschine verschlossen, eine vorgegebene Menge Reinigungsflüssigkeit in die Spülkammer eingebracht und mittels einer Umwälzpumpe umgewälzt.

Die DE 24 60 065 C2 beschreibt eine Spülmaschine zum Spülen sanitärer Behälter, bei der die Spülkammer über einen Geruchverschluß mit einem Abfluß verbunden ist. Ein mit der Spülkammer in Verbindung stehender erster Rohrabschnitt des Geruchverschlusses dient als Auffangreservoir und ist über eine Leitung mit einer Umwälzpumpe verbunden. Auch hier erfolgt zunächst eine Frischwasserspülung und anschließend eine Umwälzspülung, mit einer Heißwasserdesinfektion, wobei die umgewälzte Reinigungsflüssigkeit kontinuierlich mittels eines Durchlauferhitzers auf knapp 90°C erhitzt wird.

Die DE 195 14 468 C1 beschreibt eine Reinigungs- bzw. Desinfektionsmaschine für medizinische Sekretbehälter, die in verschlossenem Zustand in die Maschine eingelegt werden und nach Schließen der Tür automatisch geöffnet werden. Das Reinigen kann dabei mit einer Reinigungslauge und Frischwasser und das Desinfizieren mit chemischen Desinfektionsmitteln und/oder Wasserdampf erfolgen. Zum zügigen Entleeren der Spülkammer kann in einem Abflußkanal eine Abwasserpumpe vorgesehen sein.

Aus der DE 195 14 467 A1 ist ein Verfahren und eine Vorrichtung zum Reinigen und Desinfizieren von Behältern, insbesondere Urinbeuteln, bekannt. Die dort gezeigte Vorrichtung besteht aus einem Spülbehälter, in den die Urinbeutel eingebracht werden und der Sprühdüsen für die Zufuhr von Reinigungs- und Desinfektionsflüssigkeit aufweist sowie ein Absperrventil, mit dem der Spülbehälter gegenüber einem damit verbundenen Abflußkanal absperrbar ist. Bei geschlossenem Absperrventil werden die Urinbeutel aufgeschnitten und Desinfektionsflüssigkeit zugeführt, wobei sich die austretende Flüssigkeit mit dem Desinfektionsmittel mischt und das Gemisch zunächst im Spülbehälter gehalten wird. Anschließend wird das Absperrventil geöffnet und es werden die bei derartigen Spülvorrichtungen üblichen Reinigungs- bzw. Desinfektionsschritte durchlaufen. Üblich ist es hierbei, bei offenem Abflußkanal ständig frische Reinigungsflüssigkeit aus einem Vorratsbehälter in den Spülbehälter zu spritzen, die kontinuierlich abgeführt wird, was als "Frischwasserspülen" bezeichnet werden kann. Zur Erreichung guter Reinigungsergebnisse werden bei derartigen Spülmaschinen große Flüssigkeitsmengen bzw. große Vorratstanks benötigt.

In der DE 40 03 987 C2 ist eine Spülvorrichtung zum Reinigen von Endoskopen beschrieben, die einen Spülbehälter zur Aufnahme der Endoskope aufweist. Ein Flüssigkeitsauslaß des Spülbehälters steht über einer Abwasserpumpe mit einem Abflußkanal in Verbindung, wobei nach der Abwasserpumpe ein Absperrventil angeordnet ist. Ferner ist der Auslaß über eine Umwälzpumpe mit einem Sprühkopf verbunden. Derartige Umwälzspülmaschinen sind zum Reinigen und Desinfizieren von Steckbecken bzw. Urin- oder Absaugflaschen nicht geeignet, da die in diesen Spülmaschinen verwendeten Ventile und Unwälzpumpen durch den Stuhl, Urin und Schleim sowie Papierreste schnell verstopfen würden.

Aufgabe der Erfindung ist es daher, ein Verfahren und eine Vorrichtung zur Reinigung und Desinfizierung zu schaffen, die gute Reinigungsergebnisse liefert, einen geringen Wasserverbrauch hat und die oben genannten Probleme überwindet.

Das Grundprinzip der Erfindung besteht darin, bei geöffnetem Abflußkanal zunächst eine Entleerung bzw. eine Vorspülung der zu reinigenden Gegenstände durchzuführen und anschließend bei geschlossenem Abflußkanal eine Reinigung bzw. Desinfizierung im Umwälzbetrieb durchzuführen. Hierfür ist im Abflußkanal ein Absperrorgan vorgesehen, das beispielsweise ein Absperrschieber, eine Absperrkappe oder ein Absperrventil sein kann. Während der Vorspülphase ist das Absperrorgan geöffnet und die zugeführte Reinigungs- bzw. Desinfektionsflüssigkeit wird sofort abgeführt. Anschließend wird das Absperrorgan geschlossen und Reinigungs- bzw. Desinfektionsmittel wird in den Spülbehälter eingebracht. Das Flüssigkeitsgemisch wird dann über eine Umwälzpumpe umgewälzt, d. h. aus einem "Flüssigkeitssumpf" angesaugt und kontinuierlich über Sprühdüsen oder dergleichen auf die zu reinigenden Gegenstände gespritzt.

Das erfindungsgemäße Verfahren bzw. die erfindungsgemäße Vorrichtung vereinigt die Vorteile des Frischwasserspülens und des Umwälzspülens, wodurch gute Reinigungsergebnisse bei geringem Wasserverbrauch möglich sind. Grobverunreinigungen werden nämlich während der relativ kurzen Vorspülphase abgeführt und eine Fertigreinigung bzw. Desinfizierung erfolgt im wassersparenden Umwälzbetrieb.

Eine weitere Verbesserung der Reinigungsergebnisse ist durch Erhitzen der Reinigungs- bzw. Desinfektionsflüssigkeit während des Umwälzbetriebs möglich.

Nach einer Weiterbildung der Erfindung weist die Spülvorrichtung ein Sieb auf, das während des Umwälzbetriebs in den Strömungsweg zwischen dem Spülbehälter und dem Abfluß gebracht wird, wodurch verhindert wird, daß während der Umwälzung abgewaschene Teile bzw. Bauelemente der zu reinigenden Gegenstände in das Abwasser gelangen.

Nach einer Weiterbildung der Erfindung kann die Maschine in verschiedenen Betriebsarten betrieben werden. Beispielsweise kann nur die Desinfektionsphase durchlaufen werden, was z. B. für nur wenig verunreinigte medizinische Instrumente ausreichend ist, wobei die Desinfektion wahlweise chemisch oder thermisch erfolgen kann. Bei einer chemischen Desinfektion werden entsprechende Desinfektionsmittel zugeführt, während bei einer thermischen Desinfektion Keime, Bakterien bzw. Viren durch ausreichend hohe Temperaturen abgetötet werden. Ferner können in der Maschinensteuerung Betriebsprogramme implementiert sein, die wahlweise eine Nutzung der Spülvorrichtung als Steckbeckenspüler/Kombinationsmaschine, d. h. mit Vorspül- und Reinigungs- bzw. Desinfektionsphase, ermöglichen oder die einen Betrieb als Spezialmaschine zur Thermodesinfektion, gestattet, wobei unterschiedliche Reinigungstemperaturverläufe möglich sind.

Für eine thermische Desinfektion kann ein Verdampfer vorgesehen sein, der Heißdampf erzeugt, welcher in den Spülbehälter eintritt. Zusätzlich kann eine Heizeinrichtung vorgesehen sein, die während des Umwälzbetriebs die umgewälzte Reinigungs- bzw. Desinfektionsflüssigkeit erhitzt.

Nach einer Weiterbildung weist die Spülvorrichtung eine Wasserstrahlpumpe auf, die während einer thermischen Desinfektion Wasserdampf aus dem Spülbehälter abführt und einen Druckausgleich sicherstellt. Alternativ dazu kann zum Abführen des Wasserdampfes auch eine elektrisch angetriebene Pumpe verwendet werden.

Vorzugsweise sind sämtliche wasserführenden Bauteile der Spülvorrichtung während des Betriebs in den Desinfektionsvorgang eingebunden und werden somit regelmäßig desinfiziert, was die Gefahr einer Biofilmbildung bzw. Keimrückstände vermeidet.

Im folgenden wird die Erfindung anhand eines Ausführungsbeispiels im Zusammenhang mit der Zeichnung näher erläutert.
- Fig. 1: zeigt die Grundkomponenten einer Spülvorrichtung gemäß der Erfindung in schematischer Darstellung;
- Fig. 2: zeigt die Spülvorrichtung der Fig. 1 mit weiteren Komponenten;
- Fig. 3: zeigt die Sieb/Schiebervorrichtung der Fig. 1 in vergrößerter Darstellung;
- Fig. 4: zeigt ein Ausführungbeispiel der Wasserstrahlpumpe der Fig. 1 und 2 und
- Fig. 5: zeigt eine Spülmaschine mit von außen zugänglicher Wasseraufbereitungseinrichtung.

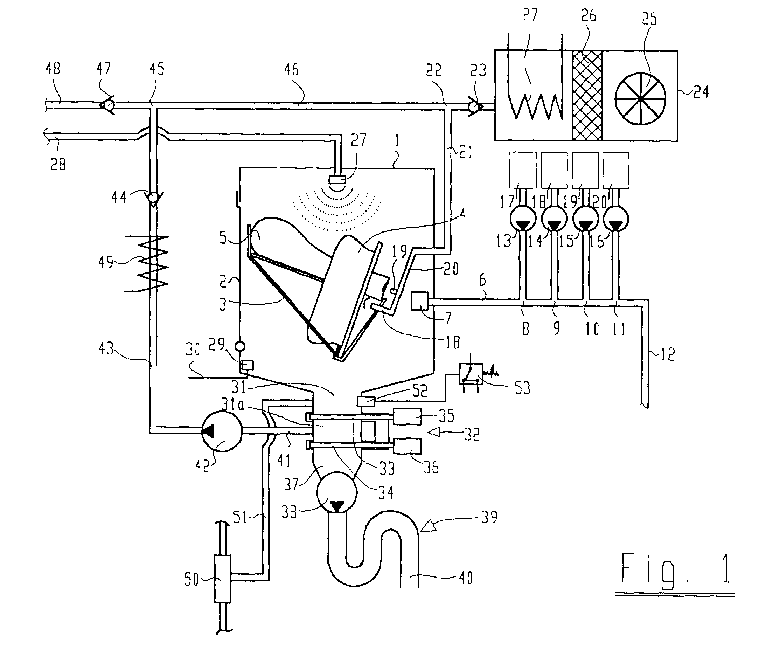

Die in Fig. 1 gezeigte Spülvorrichtung weist einen Spülbehälter 1 mit einer schwenkbaren Türe 2 auf. Im Spülbehälter 1 ist ein Haltegestell 3 angeordnet, in das ein zu reinigendes Steckbecken 4 und eine zu reinigende Urinflasche 5 eingelegt sind. Das Haltegestell 3 kann an der Innenseite des Spülbehälters 1 oder an der schwenkbaren Türe 2 befestigt sein, was ein "automatisches" entleeren des Steckbeckens 4 und der Urinflasche 5 beim Schließen der Türe 2 ermöglicht.

Ferner ist eine in das Innere des Spülbehälters 1 führende Leitung 6 vorgesehen, an deren Ende ein Einlaßventil 7 angeordnet ist, über das Reinigungs- bzw. Desinfektionsflüssigkeit in den Spülbehälter 1 eingebracht werden kann. Die Leitung 6 steht über Rohrverzweigungen 8-11 mit einer Frischwasserleitung 12 und mehreren Dosierpumpen 13-16 in Verbindung, die jeweils an einen zugeordneten Vorratsbehälter 17-20 für konzentrierte Reinigungs- oder Desinfektionslösung angeschlossen sind.

Im Spülbehälter 1 sind ferner Sprühdüsen 18 und 19 angeordnet, über die Reinigungs- bzw. Desinfektionsflüssigkeit in das Innere des Steckbeckens 4 bzw. der Urinflasche 5 gesprüht werden kann. Es können selbstverständlich mehrere solcher Sprühdüsen im Inneren des Spülbehälters 1 angeordnet sein, beispielsweise an den Seitenwänden oder am Boden, so daß sämtliche Stellen des zu reinigenden Steckbeckens bzw. der zu reinigenden Urinflasche und sämtliche Stellen des Innenraums des Spülbehälters 1 von Flüssigkeitsstrahlen erfaßt werden, wodurch eine kontinuierliche Selbstreinigung der Spülvorrichtung erreicht wird. Die Sprühdüsen 18 und 19 stehen über Leitungen 20 und 21 mit einer Rohrverzweigung 22 in Verbindung, an die über ein Rückschlagventil 23 eine Trocknungseinrichtung 24 angeschlossen ist, mit der heiße Trocknungsluft erzeugt werden kann, die über die Sprühdüsen 18 und 19 in das Innere des Spülbehälters 1 eintritt und das Steckbecken 4 und die Urinflasche 5 während einer Trocknungsphase trocknet. Die Trocknungseinrichtung 24 besteht aus einem Lüfter 25, der Luft ansaugt und diese durch ein Sterilfilter 26 sowie eine Heizeinrichtung 27 in das Innere des Spülbehälters 1 bläst.

Im oberen Bereich des Innenraums des Spülbehälters 1 ist ferner eine Düse 27 vorgesehen, die an eine Dampfleitung 28 angeschlossen ist, über die Heißdampf zugeführt werden kann. Zur Überwachung der Temperatur im Innenraum des Spülbehälters 1 ist ein Temperaturfühler 29 vorgesehen, der ein der gemessenen Temperatur entsprechendes elektrisches Signal erzeugt, das an einer elektrischen Leitung 30 abgreifbar ist.

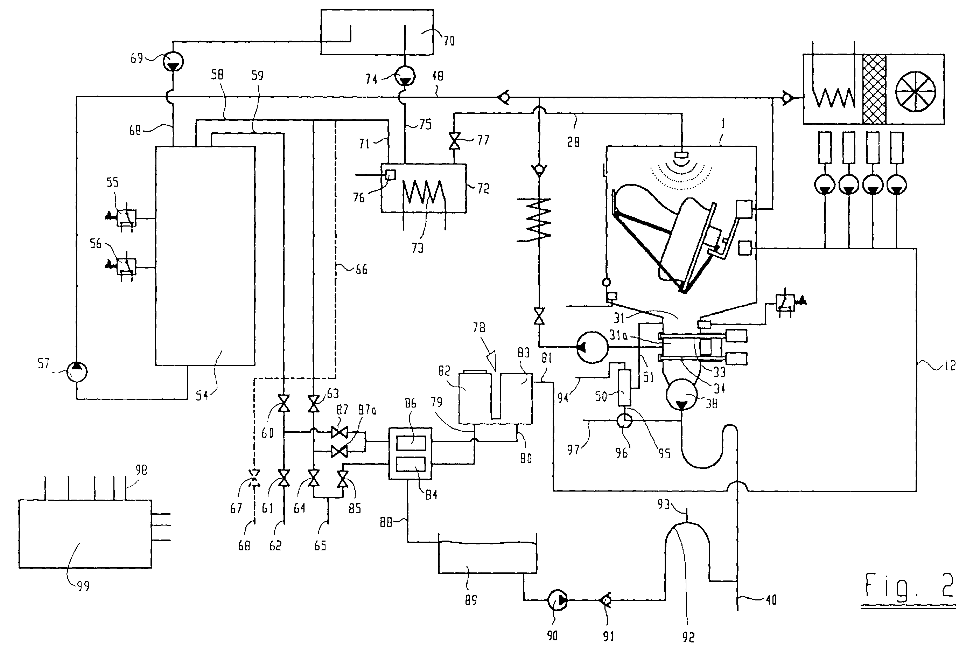

Im unteren Bereich des Spülbehälters 1 ist eine Auslaßöffnung 31 vorgesehen, die mit einem Auslaßrohr 31a verbunden ist, das eine Sieb/Absperreinrichtung 32 aufweist. Die Sieb/Absperreinrichtung besteht aus einem Sieb 33 und einem Absperrorgan 34. In der gezeigten Stellung ist das Absperrorgan 34 geschlossen und das Sieb 33 "überdeckt" das Auslaßrohr 31a, wodurch verhindert wird, daß abgewaschene Teile des Steckbeckens 4 bzw. der Urinflasche 5 in das Auslaßrohr 31a gelangen. Das Sieb 33 ist mit einer Betätigungseinrichtung 35 verbunden, die beispielsweise ein elektrisch ansteuerbarer Spindelantrieb sein kann und die ein "Herausschieben" des Siebes 33 aus dem Auslaßrohr 31a ermöglicht. Bei herausgeschobenem Sieb 32 besteht dann eine direkte Strömungsverbindung zwischen dem Innenraum des Spülbehälters 1 und dem Auslaßrohr 31a, was ein ungehindertes Abfließen von Stuhl- und Papierresten ermöglicht. Das Absperrorgan 34 ist ebenfalls über eine zugeordnete Betätigungseinrichtung 36 öffenbar bzw. absperrbar. Wie im Zusammenhang mit Fig. 2 näher erläutert, kann das Absperrorgan 34 beispielsweise ein Absperrschieber sein oder eine schwenkbare Absperrkappe oder ein Absperrventil. Das Absperrorgan 34 ist über eine Verbindungsleitung 37 mit einer elektrisch betriebenen Abwasserpumpe 38 und diese über einen Syphon 39 mit einen Abflußkanal 40 verbunden.

Das Auslaßrohr 31a steht ferner über einen Stichkanal 41 mit einer elektrisch betriebenen Umwälzpumpe 42 in Verbindung. Von der Umwälzpumpe 42 führt eine Leitung 43, in der ein Rückschlagsventil 44 angeordnet ist, zu einer Rohrverzweigung 45, die über eine Leitung 46 mit der Rohrverzweigung 22 in Verbindung steht. Die Rohrverzweigung 45 steht zusätzlich über ein Rückschlagventil 47 und über eine Leitung 48 mit einem Wasserspeicher (Fig. 2) in Verbindung. Ferner ist eine elektrisch ansteuerbare Heizeinrichtung 49 vorgesehen, die ein Erhitzen der durch die Leitung 43 strömenden Flüssigkeit ermöglicht.

Weiterhin weist die Spülvorrichtung eine Wasserstrahlpumpe 50 auf, die im Zusammenhang mit den Fig. 2 und 4 näher erläutert wird und die über eine Saugleitung 51 mit dem Auslaßrohr 31a in Verbindung steht. Die Wasserstrahlpumpe 50 ist dazu vorgesehen, während einer thermischen Desinfektionsphase Wasserdampf aus dem Innern des Spülbehälters abzusaugen und eine gute Verteilung des Dampfes und einen Druckausgleich sicherzustellen. Im unteren Bereich des Spülbehälters 1 ist ferner ein Wasserstandssensor 52 vorgesehen, der bei geschlossenem Absperrorgan 34 den Flüssigkeitspegel im Auslaßrohr 31a bzw. im unteren Bereich des Spülbehälters 1 mißt, ab einem bestimmten Flüssigkeitspegel ein elektrisches Signal erzeugt und einen Niveauschalter 53 betätigt, der die Zufuhr weiterer Reinigungs- bzw. Desinfektionsflüssigkeit abschaltet.

Fig. 2 zeigt eine detailliertere Darstellung der Spülvorrichtung der Fig. 1, in der weitere Komponenten dargestellt sind.

Es ist ein Wasserspeicher 54 zu erkennen, dessen Wasserstand durch Niveauschalter 55 und 56 überwacht wird und der über eine Förderpumpe 57 mit der zum Spülbehälter 1 führenden Leitung 48 in Verbindung steht. Der Wasserspeicher 54 kann über Leitungen 58 bzw. 59 mit Wasser befüllt werden, wobei die Leitung 59 über Ventile 60 und 61 mit einem Warmwasseranschluß 62 in Verbindung steht. Die Leitung 58 steht über Ventile 63 und 64 mit einem Kaltwasseranschluß 65 und optional über eine Leitung 66 und ein Ventil 67 mit einem Anschluß 68 für vollentsalztes Wasser (VE-Wasser) in Verbindung, was durch gestrichelte Linien dargestellt ist. Der Wasserspeicher 54 weist ferner eine Leitung 68 auf, über die mittels einer Dosierpumpe 69 Klarspüler und Entkalkerflüssigkeit aus einem Vorratsbehälter 70 zugeführt werden kann.

Weiterhin ist die Leitung 58 über eine Leitung 71 mit einem Verdampfer 72 verbunden, der eine integrierte Heizeinrichtung 73 zur Erzeugung von Heißdampf aufweist. Dem Verdampfer 72 kann über die Leitung 71 Wasser zugeführt werden und mittels einer Dosierpumpe 74 kann über eine Leitung 75 Klarspüler und Entkalkerflüssigkeit aus dem Vorratsbehälter 70 beigemischt werden. Ferner ist ein Temperaturfühler 76 vorgesehen, der die Temperatur im Verdampfer 72 mißt. Der erzeugte Heißdampf kann über ein Ventil 77, die Dampfleitung 28 und die Düse 27 in das Innere des Spülbehälters 1 einströmen.

Zusätzlich ist eine Wasserenthärtereinrichtung 78 vorgesehen, die das über die Frischwasserleitung 12 dem Spülbehälter 1 zugeführte Wasser enthärtet. Die Wasserenthärtereinrichtung 78 weist einen Kaltwassereingang 79, einen Warm/Heißwassereingang 80 und einen Ausgang 81 auf, der mit der Frischwasserleitung 12 verbunden ist. Das Kaltwasser strömt zunächst in eine erste Kammer 82, die beispielsweise mit einem Enthärtersalz gefüllt ist und das Warmwasser strömt in eine zweite Kammer 83, die mit einer Levatit-Masse gefüllt sein kann. Der Kaltwassereingang 79 der Wasserenthärtereinrichtung 78 steht über ein Unterdruckventil 84 und ein Ventil 85 mit dem Kaltwasseranschluß 65 in Verbindung und der Warm/Heißwassereingang 80 ist über ein Unterdruckventil 86 und Mischventile 87 und 87a mit dem Warmwasseranschluß 62 bzw. mit dem Kaltwasseranschluß 65 verbunden. Die Unterdruckventile 84 bzw. 86 bilden eine sogenannte "freie Fließstrecke" und führen Wasser, das von der Wasserenthärtereinrichtung 78 in Richtung zum Warmwasseranschluß 62 bzw. zum Kaltwasseranschluß 65 zurückfließt über eine Leitung 88 einem Sammelbehälter 89 zu. Das Wasser im Sammelbehälter 89 kann mittels einer Abwasserpumpe 90 über ein Rückschlagventil 91 und einen Syphon 92, der eine Entlüftung 93 aufweist, in den Abflußkanal 40 gepumpt werden.

Ferner ist zu erkennen, daß die Wasserstrahlpumpe 50, deren Saugleitung 51 mit dem Auslaßrohr 31a in Verbindung steht, einen Zufluß 94 und einen Abfluß 95 aufweist. Der Zufluß 94 kann beispielsweise über ein Ventil mit dem Kaltwasseranschluß 65 oder mit einer separaten externen Wasserzuleitung verbunden sein und der Abfluß 95 kann über ein Umschaltventil 96, das sich hier in einer ersten Stellung befindet, mit dem Abflußkanal 40 verbunden sein. In einer anderen Stellung des Umschaltventils 96 ist der Abfluß 95 mit einer Leitung 97 verbunden, die in den Wasserspeicher 54 geführt sein kann.

Alternativ zu der Wasserstrahlpumpe 50 kann auch eine elektrisch angetriebene Pumpe verwendet werden.

Sämtliche "Komponenten" der Spülvorrichtung, die elektrisch ansteuerbar sind, wie z.B. die Dosierpumpen 13-16 (Fig. 1), die Trocknungseinrichtung 24, das Sieb 33, das Absperrorgan 34, die Umwälzpumpe 42, die Heizeinrichtung 49, die Förderpumpe 57, die Ventile 60, 61, 63, 64, 67, 87, 87a, die Abwasserpumpe 38, etc. sind über elektrische Steuerleitungen 98 mit einer zentralen Steuervorrichtung 99 verbunden. Die Steuervorrichtung 99 kann beispielsweise ein in die Spülvorrichtung intergrierter Mikrorechner sein, der die oben genannten "Komponenten" der Spülvorrichtung gemäß unterschiedlicher vom Benutzer auswählbarer Spülprogramme steuert.

Im folgenden wird auf mögliche Spülprogramme eingegangen.

Bei einem ersten Spülprogramm wird zum Reinigen bzw. Desinfizieren von Steckbecken zunächst eine Vorspül- bzw. Entleerungsphase durchgeführt, in der das Sieb 33 aus dem Auslaßrohr 31a herausgeschoben ist und das Absperrorgan 34 offen ist, wobei aus dem in die Haltevorrichtung 3 eingelegten Steckbecken 4 bzw. aus der Urinflasche 5 der Inhalt auslaufen kann und durch die Abwasserpumpe 38 in den Abflußkanal 40 gepumpt wird. Gleichzeitig werden das Steckbecken 4 bzw. die Urinflasche 5 über die Sprühdüsen 18 und 19 mit Reinigungsflüssigkeit besprüht, die über die Leitung 58 und die Förderpumpe 57 aus dem Wasserspeicher 54 zugeführt wird. Während dieser "Vorreinigungsphase" arbeitet die Spülvorrichtung als "Frischwasserspülmaschine", wobei ständig frische Reinigungsflüssigkeit in den Spülbehälter 1 gespritzt wird und bei offenem Absperrorgan 34 durch die Abwasserpumpe 38 abgepumpt wird.

Nach Beendigung der Vorspül- bzw. Entleerungsphase schaltet die Spülvorrichtung in eine "Umwälzphase" um, in der das Steckbecken 4 bzw. die Urinflasche 5 fertiggereinigt und desinfiziert werden. Während der Umwälzphase ist das Sieb 33 in das Auslaßrohr 31a geschoben und das Absperrorgan 34 ist geschlossen. Über das Einlaßventil 7 wird eine gewisse Menge an Reinigungs- bzw. Desinfektionsflüssigkeit in den Spülbehälter eingebracht, die durch Mischen von über die Leitung 12 zugeführtem Frischwasser mit Reinigungs- bzw. Desinfektionslösung entsteht, die in den Vorratsbehältern 17-20 enthalten ist. Es wird solange Reinigungs- bzw. Desinfektionsflüssigkeit in den Spülbehälter 1 eingebracht, bis im Auslaßrohr 31a ein bestimmter Flüssigkeitspegel erreicht wird, der durch den Wasserstandssensor 52 festgestellt wird. Das Auslaßrohr 31a bildet somit ein "Sumpfreservoir", das während der Umwälzphase mit Reinigungs- bzw. Desinfektionsflüssigkeit gefüllt ist. Die in diesem Reservoir enthaltene Reinigungs- bzw. Desinfektionsflüssigkeit wird durch die Umwälzpumpe 42 über den Stichkanal 41 und die Leitungen 43, 46, 21 und 20 ständig umgewälzt, und kann dabei durch die Heizeinrichtung 47 erhitzt werden.

In einer anderen Betriebsart wird die Spülvorrichtung als "Thermodesinfektions-Spülmaschine" eingesetzt. Hierbei kann Heißdampf, der im Verdampfer 72 erzeugt wird, über die Dampfleitung 28 und die Düse 27 in den Spülbehälter 1 eingebracht werden. Bei dieser Betriebsart saugt die Wasserstrahlpumpe 50 über die Saugleitung 51 Heißdampf aus dem Innenraum des Spülbehältes 1 ab, wodurch ein Druckausgleich im Spülbehälter 1 sichergestellt ist und eine gleichmäßige Verteilung des Dampfes erreicht wird. Beim Betrieb der Wasserstrahlpumpe 50 ist deren Zufluß 94 an den Kaltwasseranschluß 65 angeschlossen und das zugeführte Wasser bzw. der angesaugte Dampf werden in den Abflußkanal 40 oder in den Wasserspeicher 54 gepumpt. Das Befüllen des Wasserspeichers 54 bzw. des Verdampfers 72 und die Frischwasserzufuhr über die Frischwasserleitung 12 werden ebenfalls durch die Steuervorrichtung 99, d.h. durch Ansteuern der einzelnen Ventile gesteuert.

In der Steuereinrichtung 99 können weitere Reinigungs- bzw. Desinfektionsprogramme implementiert sein, die sich beispielsweise durch die Länge einzelner Reinigungs- bzw. Desinfektionsphasen, die zugeführten Mengen an Reinigungs- bzw. Desinfektionsflüssigkeit bzw. der zugeführten Klarspüler und Entkalkermengen sowie durch die Stellungen des Siebes 33 und des Absperrorgans 11 unterscheiden können.

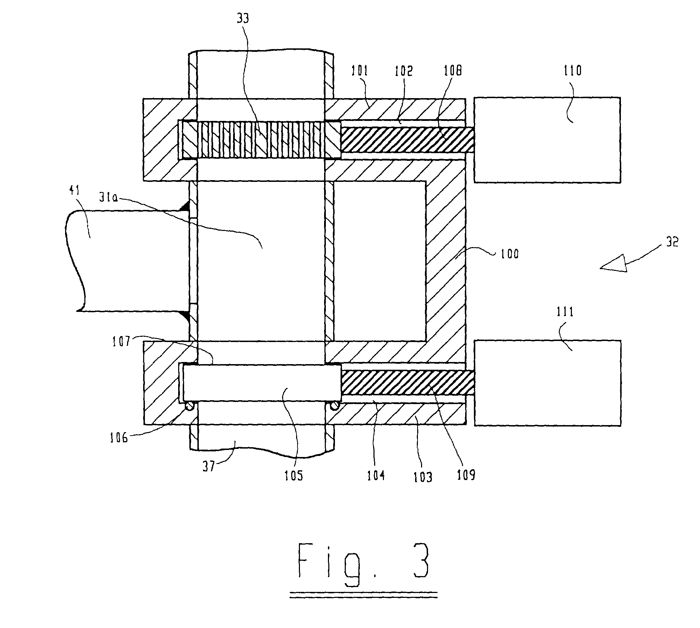

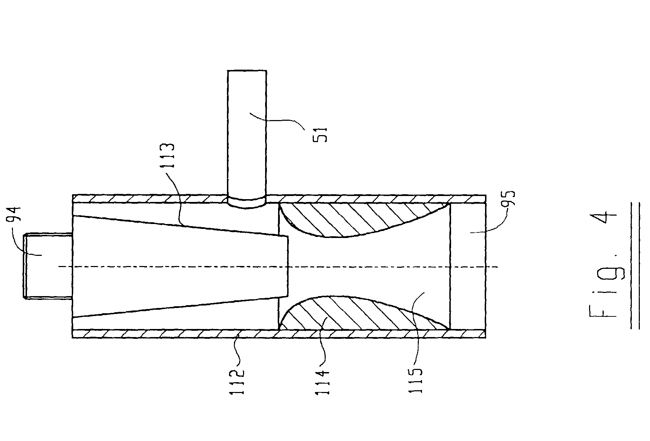

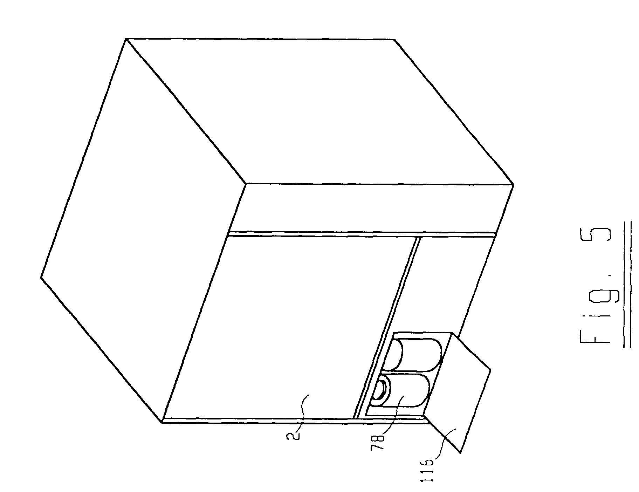

Fig. 3 zeigt eine vergrößerte Darstellung der in den Fig. 1 und 2 gezeigten der Sieb/Absperreinrichtung 32. Die Sieb/Absperreinrichtung 32 weist ein Gehäuse 100 auf, das in der hier gezeigten Querschnittsansicht U-förmig ist, wobei in einem ersten Gehäuseschenkel 101 ein Verschiebeschlitz 102 vorgesehen ist, in dem das Sieb 33 verschieblich geführt ist. In einem zweiten Gehäuseschenkel 103 ist ebenfalls ein Verschiebeschlitz 104 vorgesehen, in dem das Absperrorgan verschieblich geführt ist, das hier als Absperrschieber 105 ausgeführt ist. Zur Abdichtung des Auslaßrohres 31a bzw. des Stichkanals 41 gegenüber der mit dem Abflußkanal 40 (Fig. 1) verbundenen Verbindungsleitung 37 ist eine elastische O-Ring-Dichtung 106 vorgesehen, die in eine Ausnehmung des zweiten Verschiebeschlitzes 104 des zweiten Gehäuseschenkels 103 eingesetzt ist und die gegen den Absperrschieber 105 drückt. Zusätzlich kann eine weitere O-Ring-Dichtung auf einer dem O-Ring 106 gegenüberliegenden Seite 107 des Absperrschiebers 105 vorgesehen sein.

Das Sieb 33 und der Absperrschieber 105 können über zugeordnete Betätigungsspindeln 108 bzw. 109 und zugeordnete Spindelantriebe 110 bzw. 111 in den Verschiebeschlitzen 102 bzw. 104 verschoben werden.

Alternativ zu dem gezeigten Absperrschieber kann auch eine Absperrkappe oder ein Absperrventil verwendet werden.

Fig. 4 zeigt ein Ausführungsbeispiel einer Wasserstrahlpumpe mit einem Gehäuse 112, das den Zufluß 94, den Abfluß 95 und die Saugleitung 51 aufweist. Am Zufluß 94 ist eine sich gehäuseeinwärts verjüngende konische Düse 113 vorgesehen, durch die das eintretende Wasser beschleunigt wird. Die Düse 113 mündet in eine axial dazu versetzte zweite Düse 114, die einen Diffusor 115 aufweist. Durch die Beschleunigung des Wassers entsteht in der Saugleitung 51. ein Unterdruck, der eine Ausaugung von Heißdampf aus Spülbehälter 1 (Fig. 2) ermöglicht. Gleichzeitig kühlt sich das Wasser im Diffusor 115 ab und kann - nachdem es über den Abfluß 95 abgeführt wurde - zur Kühlung einzelner Komponenten der Spülvorrichtung oder anderer Maschinen verwendet werden.

Fig. 5 zeigt eine Außenansicht einer Spülmaschine, bei der unterhalb der Türe 2 ein Wartungsdeckel 116 vorgesehen ist, der einen Zugang zur Wasserenthärtereinrichtung 78 ermöglicht. Über den Wartungsdeckel 116 kann die Wasserenthärtereinrichtung entnommen und das Enthärtermittel ausgetauscht werden.

## Patentansprüche

1. Verfahren zum Reinigen und Desinfizieren eines Verunreinigungen enthaltenden Gefäßes, insbesondere eines Steckbeckens, einer Urinflasche oder einer Absaugflasche, das zur Reinigung und Desinfizierung in eine Spülkammer einer Spülvorrichtung eingebracht wird, mit den Schritten:
a) Entleeren und Vorspülen des Gefäßes (4, 5) durch kontinuierliches Besprühen mit frisch zugeführter Reinigungsflüssigkeit und kontinuierliches Abführen verunreinigter Reinigungsflüssigkeit über einen Abfluß (31, 31a, 34, 37-40) der Spülvorrichtung;
b) Unterbrechen des Besprühens mit Reinigungsflüssigkeit bei weiterem Abführen verunreinigter Reinigungsflüssigkeit;
c) Verschließen des Abflußes (31a) der Spülvorrichtung;
d) Einbringen einer vorbestimmten Menge an Reinigungs- bzw. Desinfektionsflüssigkeit in die Spülkammer (1);
e) Kontinuierliches Reinigen und Desinfizieren des Gefäßes (4, 5) durch Umwälzen und Besprühen mit der in der Spülkammer (1) enthaltenen Reinigungs- bzw. Desinfektionsflüssigkeit
**dadurch gekennzeichnet,**
f) daß in die Spülkammer (1) Heißdampf eingebracht wird und
g) daß beim Einbringen des Heißfdampfes mittels einer Wasserstrahlpumpe (50) bzw. einer elektrisch angetriebenen Pumpe feuchte Luft aus der Spülkammer (1) abgesaugt wird.

2. Verfahren nach Anspruch 1,
**dadurch gekennzeichnet,**
daß die umgewälzte Reinigungs- bzw. Desinfektionsflüssigkeit während des Umwälzens erhitzt wird.

3. Verfahren nach einem der Ansprüche 1 oder 2,
**dadurch gekennzeichnet,**
daß der Abfluß (31) der Spülvorrichtung während des Vorspülens ganz geöffnet ist und während des Umwälzens durch ein Sieb (33) überdeckt ist.

4. Verfahren nach einem der Ansprüche 1 bis 3,
**dadurch gekennzeichnet,**
daß die Reinigungs- bzw. Desinfektionsflüssigkeit in der Spülvorrichtung durch Mischen von Wasser und Reinigungs- bzw. Desinfektionslösung hergestellt wird.

5. Vorrichtung zum Reinigen und Desinfizieren eines Verunreinigungen enthaltenden Gefäßes, insbesondere eines Steckbeckens, einer Urinflasche oder einer Absaugflasche, mit
a) einer Spülkammer, in die das zu reinigende Gefäß einbringbar ist,
b) einem Auslaß der Spülkammer, der mit einem Abflußkanal in Strömungsverbindung bringbar ist,
c) einem Absperrorgan, das zwischen dem Auslaß und dem Abflußkanal angeordnet ist,
d) einem zwischen dem Auslaß und dem Absperrorgan vorgesehenen Auffangreservoir zum Auffangen von Reinigungs- bzw. Desinfektionsflüssigkeit,
e) einer Umwälzpumpe, die saugseitig mit dem Auffangreservoir und druckseitig mit in die Spülkammer ragenden Sprühelementen verbunden ist
**dadurch gekennzeichnet,**
f) daß eine Abwasserpumpe (38) zum Abpumpen verunreinigter Reinigungsflüssigkeit vorgesehen ist, die zwischen dem Absperrorgan (34) und dem Abflußkanal (40) angeordnet ist,
g) daß ein Heißdampferzeuger (72) vorgesehen ist, der mit den in die Spülkammer (1) ragenden Sprühelementen (18, 19) in Strömungsverbindung steht und
h) daß eine Wasserstrahlpumpe (50) oder eine elektrisch angetriebene Pumpe vorgesehen ist, die einen Sauganschluß (51) aufweist, der mit der Spülkammer (1) in Verbindung steht.

6. Vorrichtung nach Anspruch 5,
**dadurch gekennzeichnet,**
daß das Absperrorgan (34) ein Absperrschieber (105) ist.

7. Vorrichtung nach Anspruch 5,
**dadurch gekennzeichnet,**
daß das Absperrorgan (34) eine Absperrkappe ist.

8. Vorrichtung nach Anspruch 5,
**dadurch gekennzeichnet,**
daß das Absperrorgan (34) ein Absperrventil ist.

9. Vorrichtung nach einem der Ansprüche 5 bis 8,
**dadurch gekennzeichnet,**
daß das Absperrorgan (34) eine Dichtung (106) zur Abdichtung des Auffangreservoirs (31a) gegenüber der Abwasserpumpe (38) aufweist.

10. Vorrichtung nach Anspruch 9,
**dadurch gekennzeichnet,**
daß die Dichtung (106) eine O-Ring-Dichtung ist.

11. Vorrichtung nach einem der Ansprüche 5 bis 10,
**dadurch gekennzeichnet,**
daß ein Sieb (33) vorgesehen ist, das durch eine Betätigungseinrichtung (35) in den zwischen dem Auslaß (31) und dem Absperrorgan (34) liegenden Strömungsweg bringbar ist, wobei das Sieb (33) in diesem Strömungsweg liegt, wenn das Absperrorgan (34) geschlossen ist.

12. Vorrichtung nach einem der Ansprüche 5 bis 11,
**dadurch gekennzeichnet,**
daß ein druckseitiger Anschluß (95) der Wasserstrahlpumpe (50) bzw. der elektrisch angetriebenen Pumpe mit einer zu kühlenden Einrichtung in Verbindung steht.

13. Vorrichtung nach einem der Ansprüche 5 bis 12,
**dadurch gekennzeichnet,**
daß eine Heizvorrichtung (49) zum Aufheizen der von der Umwälzpumpe (42) umzuwälzenden Reinigungs- bzw. Desinfektionsflüssigkeit vorgesehen ist.

14. Vorrichtung nach einem der Ansprüche 5 bis 13,
**dadurch gekennzeichnet,**
daß ein Wasserspeicher (54) vorgesehen ist, der mit in die Spülkammer mündenden Sprühelementen (18, 19) in Strömungsverbindung steht.

15. Vorrichtung nach einem der Ansprüche 5 bis 14,
**dadurch gekennzeichnet,**
daß eine Wasserenthärtereinrichtung (78) vorgesehen ist, die der Spülkammer (1) zugeführtes Wasser enthärtet.
